# EUROPEAN PATENT SPECIFICATION

(11) **EP 1 708 738 B1**
(45) Date of publication and mention of the grant of the patent: **04.05.2016**
(21) Application number: 05711314.4
(22) Date of filing: 10.01.2005
(51) Int. Cl.: A61K 38/28

(54) **A METHOD OF REDUCING SERUM PROINSULIN LEVELS IN TYPE 2 DIABETICS**
VERFAHREN ZUR VERRINGERUNG DES PROINSULINSPIEGELS IM SERUM BEI ZUCKERKRANKEN MIT TYPE 2 DIABETES
PROCEDE PERMETTANT DES REDUIRE LES TAUX SERIQUES DE PROINSULINE DANS LE DIABETE DE TYPE 2

(30) Priority: 12.01.2004 US 535945 P
(43) Date of publication of application: 11.10.2006
(73) Proprietor: MannKind Corporation, Valencia, CA 91355 (US)
(72) Inventor: CHEATHAM, Wayman, Wendell, Columbia, MD 21045 (US); PFUETZNER, Andreas, 55130 Mainz (DE); BOSS, Anders Hasager, Princeton, NJ 08540 (US)
(74) Representative: Potter Clarkson LLP
(86) International application number: PCT/US2005/000596
(87) International publication number: WO 2005/067964

(56) References cited:
- PFUETZNER ET AL: ABSTRACT 812: "Influence of small dose i.v.,s.c. and pulmonary insulin treatment on prandial glucose control in patients with type 2 diabetes" INTERNET ARTICLE / VIVÍSIMO, [Online] 2001, XP002329615 37TH ANNUAL MEETING OF THE EASD, GLASGOW, 9-13 SEPTEMBER 2001 Retrieved from the Internet: URL:http://www.easd.org/customfiles/easd/3 7th/Posters01.html> [retrieved on 2005-05-26]
- CHEATHAM ET AL: "Desirable dynamics & performance of inhaled insulin compared to subcutaneous insulin given at mealtime in type 2 diabetes: A report from the Technosphere/Insulin study group" DIABETES TECHNOLOGY & THERAPEUTICS, vol. 6, April 2004 (2004-04), page 234, XP008047658
- WARREN ET AL: "Postprandial versus preprandial dosing of biphasic insulin aspart in elderly type 2 diabetes patients" DIABETES RESEARCH AND CLINICAL PRACTICE, vol. 66, October 2004 (2004-10), pages 23-29, XP004610519
- RASKIN ET AL: "Continuous subcutaneous insulin infusion and multiple daily injection therapy are equally effective in type 2 diabetes" DIABETES CARE, vol. 26, September 2003 (2003-09), pages 2598-2603, XP002321785

## Description

### FIELD OF THE INVENTION

This invention is generally in the field of treatment of diabetes mellitus, type 2 and related sequela using prandial insulin substitution regimens that mimic the meal-related first phase insulin response. In particular it relates to the reduction of serum proinsulin levels, pancreatic stress, and atherogenic factors in type 2 diabetics.

### BACKGROUND OF THE INVENTION

Diabetes mellitus is present in 17 million Americans. Its prevalence is growing at a rate of 4.5% per year, particularly diabetes mellitus type 2, also variously called adult-onset and insulin-resistant diabetes. The paradigmatic defect in diabetes is the mis-regulation of serum glucose levels. In addition to the deleterious effects of hyperglycemia, inability to properly respond to high serum glucose levels creates stress on the pancreas that can accelerate progression of the disease in type 2 diabetes. Also, the diagnosis of diabetes carries a 2 to 4 times greater risk of stroke and heart attack in individuals with the disorder. The presence of diabetes in an individual without known heart disease places their risk of new myocardial infarction at the same level as a person who has already had a myocardial infarction but who does not have diabetes. Thus there are important therapeutic goals in the treatment of type 2 diabetes in addition to hyperglycemia per se that are not adequately addressed by currently available treatments.

It is therefore an object of the present invention to provide alternative treatments, especially treatments for type 2 diabetes.

Cheatham and Pfuetzner, Diabetes Technology & Therapeutics, April 2004, Abstract, discloses a study of inhaled insulin in diabetes patients, and is not prior art for subject matter which is entitled to priority. Pfuetzner et al, 37th Annual Meeting of the EASD, 2001, discloses an exploratory study in which small doses of fast-acting preprandial insulin were administered to type 2 diabetics. Warren et al, Diabetes Research & Clinical Practice, October 2004, p23-29, discloses a study of postprandial versus preprandial dosing of biphasic insulin aspart in elderly type 2 diabetics, and is not prior art for subject matter which is entitled to priority. Pfuetzner et al, Diabetes Technology & Therapeutics, 2002, p589-594, discusses studies of the pharmacokinetics of an inhaled Technosphere®/insulin formulation. Weiss et al, Arch. Intern. Med., 2003, p2277-2282 discloses a clinical trial of an inhaled insulin formulation in patients with type 2 diabetes inadequately controlled with oral agents. Cheatham *et al,* Abstract 457-P, A novel pulmonary insulin formulation replicates first phase insulin release and reduces serum proinsulin levels, (http://professional.diabetes.org/Content/Posters/2004/p457-P.pdf) discloses a study of Technosphere®/insulin formulations administered to type 2 diabetics, and is not prior art for subject matter which is entitled to priority.

### SUMMARY OF THE INVENTION

A first aspect of the invention provides a dose of insulin that is effective to reduce serum proinsulin levels and which mimics a first phase insulin response, for use as a prandial medicament for reducing risk factors for atherosclerotic cardiovascular disease in a patient who is in the early course of type 2 diabetes.

A second aspect of the invention provides a use of a dose of insulin that is effective to reduce serum proinsulin levels and which mimics a first phase insulin response, in the manufacture of a prandial medicament for reducing risk factors for atherosclerotic cardiovascular disease in a patient who is in the early course of type 2 diabetes.

The dose of insulin is for reducing serum proinsulin levels, lessening post-prandial pancreatic stress, and reducing risk factors for atherosclerosis in subjects with diabetes mellitus, type 2. The dose of insulin is for administration in a manner that mimics the meal-related first phase insulin response, wherein the dose of insulin may be greater than 24 IU, which is sufficient to reduce serum levels of proinsulin. Mimicking first phase kinetics, peak serum insulin levels can be reached within about 18 minutes of administration. In increasingly preferred embodiments peak serum insulin levels can be reached within about 15, 12, or 10 minutes of administration. Serum insulin levels return to baseline within about two hours of administration. In one embodiment, the insulin is for administering within one hour after the start of a meal. In a preferred embodiment, the insulin is for administering within about 10 minutes after starting a meal.

Diketopiperazine microparticle drug delivery systems are used in various embodiments of the invention. In further embodiments, the insulin is for administering by pulmonary delivery using synthetic biodegradable polymeric or diketopiperazine microparticles incorporating the insulin. In preferred embodiments, delivery is achieved by inhalation of a dry powder. In aspects of the invention utilizing diketopiperazine microparticles, fumaryl diketopiperazine is a preferred type of diketopiperazine, the insulin is dimeric or monomeric, and preferred dosages are up to 90 IU of insulin. In preferred embodiments, inhalation of the dry powder is facilitated by use of a unit dose inhaler. According to the invention, risk factors for atherosclerosis may be reduced, including ones wherein the risk factor is LDL particle size and LDL particle size is increased; and wherein the risk factor is plasminogen activator inhibitor type-1 (PAI-1), and PAI-1 expression is reduced.

### BRIEF DESCRIPTION OF THE DRAWING

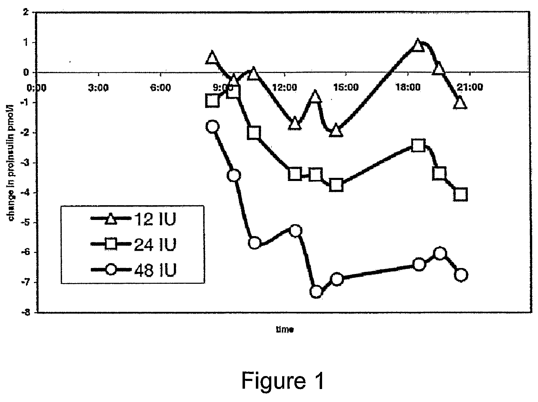

Figure 1 is a graph of the changes in proinsulin levels over time, following pulmonary administration of diketopiperazine/insulin particles.

### DETAILED DESCRIPTION OF THE INVENTION

The development of pulmonary insulin formulations is designed to provide new and effective alternatives for meal-related (prandial) insulin substitution in diabetic patients. The ideal kinetics of insulin formulations for prandial substitution include a rapid and early onset of action and a duration of action long enough to cover meal-related glucose absorption. One problem with existing formulations of insulin for subcutaneous (s.c.) injections has been the unpredictable variability of absorption, exceeding 50% in some cases, and the relatively slow rise in serum insulin levels compared to physiologic meal-related first phase insulin response, in which serum insulin levels can peak by about 6 minutes.

Meal-related first phase insulin originates from storage vesicles in the beta cells of the islets of Langerhans of the pancreas, where proinsulin undergoes enzymatic cleavage into insulin and C-peptide. Type 2 diabetes, as distinct from type 1, is characterized by a loss of the meal-related first phase insulin response. This loss occurs early in the disease process. Type 2 diabetes, again as distinct from type 1, is further characterized by elevated levels of serum proinsulin. Such circulating intact proinsulin (iPi) likely signifies that insulin requirements exceed beta cell capacity, causing pancreatic stress leading to premature release of the storage vesicles.

The rat model of type 2 diabetes has been used to see what happens if insulin is administered versus sham injections to determine how fast the cohort develop type 2 diabetes. In those animals treated with small injections of insulin, it has been shown that they take as much as twice as long to develop the prevalence of diabetes as the sham treated animals. Also, when they are sacrificed, those receiving the insulin injections have a higher number of viable beta cells in their pancreas. The accepted interpretation is that the injections of insulin take stress off the pancreas and that something about stressing the pancreas makes the beta cells die off faster. Thus serum proinsulin is a useful indicator of pancreatic stress and relief of this stress is observable as reduction in serum proinsulin levels.

Type 2 diabetes is typified by elevated serum levels of proinsulin from early points in the progression of the disease. In addition to signifying pancreatic stress, serum proinsulin can be detrimental in its own right. Serum proinsulin is positively associated with an increased risk of atherosclerotic cardiovascular disease in humans (Haffner et al., Stroke.29:1498-1503, 1998; Hanley et al., Diabetes Care 24:1240-1247, 2001; Zethelius et al., Circulation. 105:2153, 2002). It is also associated with known atherogenic risk factors such as reduced LDL particle size (Festa et al. Diabetes Care 22:1688-1693, 1999) and increased plasminogen activator inhibitor type-1 (PAI-1) expression (Schneider et al., Diabetes, 41:890-895, 1992). Administration of proinsulin to humans in clinical trials in the 1980s resulted in an increased incidence of myocardial infarction and death in subjects receiving the agent. Thus reduction of serum proinsulin levels is an additional therapeutic goal, and one that is not addressed by the current therapies used in the earlier stages of the disease that focus on serum glucose level.

### Insulin Formulations

Insulin is commercially available, in either monomeric or dimeric form.

Useful carriers are also available, or can be made using published technology. Pulmonary insulin delivered using diketopiperazine microparticles is rapidly absorbed reaching peak serum levels in about 10 to 15 minutes. This is fast enough to mimic the kinetics of the physiologic meal-related first phase insulin response, as evidenced by the shutoff of gluconeogenesis that is observed. Such treatment also leads to reduced levels of serum proinsulin, which is not seen with slower acting insulin preparations. The relative ease of administration via this mode of treatment also facilitates treatment of type 2 diabetes much earlier in the course of the disease than has been traditionally practiced. Thus by using an insulin delivery that mimics first phase kinetics, serum proinsulin levels can be reduced and the literature indicates that this will be accompanied by similar reductions in atherogenic risk factors. By commencing insulin therapy early in the course of the disease, reduction in pancreatic stress can slow progression of the disease itself.

Diketopiperazine microparticle drug delivery systems and associated methods are described in U.S. Patents 5,352,461 and 5,503,852 entitled Self Assembling Diketopiperazine Drug Delivery System and Method for Making Self Assembling Diketopiperazine Drug Delivery System, respectively. The use of diketopiperazine and biodegradable polymer microparticles in pulmonary delivery is described in U.S. Patents 6,428,771 and 6,071,497 entitled Method for Drug Delivery to the Pulmonary System, and Microparticles for Lung Delivery Comprising Diketopiperazine, respectively. Details regarding various aspects of possible formulation and manufacturing processes can be found in U.S. Patents 6,444,226 and 6,652,885 both entitled Purification and Stabilization of Peptide and Protein Pharmaceutical Agents, and in U.S. Patent 6,440,463 entitled Methods for Fine Powder Formation. The properties and design of a preferred breath-powered dry powder inhaler system is disclosed in PCT/US00/40454 and PCT/US2004/028699.

Other formulations can consist solely of drug particles, drug plus surfactant particles, and polymer drug particles, such as particles of poly(lactic acid-co-glycolic acid) encapsulating the drug to be administered.

### Method of Administration

A dose of insulin is provided for reducing serum proinsulin levels, lessening post-prandial pancreatic stress, and reducing risk factors for atherosclerosis in subjects with diabetes mellitus, type 2. Insulin is for administering in a manner that mimics the meal-related first phase insulin response. A dose of insulin of greater than 24 IU, which is sufficient to reduce serum levels of proinsulin, is provided. The insulin administration is commenced early in the course of the disease. Mimicking first phase kinetics, peak serum insulin levels can be reached within about 18 minutes of administration. Formulations and methods of administration, preferably by pulmonary administration, are selected so that peak serum insulin levels can be reached within about 15, 12, or 10 minutes of administration. Serum insulin levels return to baseline within about two hours of administration. In one embodiment, insulin is for administering within one hour after the start of a meal. In a preferred embodiment, insulin is for administeringed within about 10 minutes after starting a meal. According to the invention, risk factors for atherosclerosis may be reduced, including ones wherein the risk factor is LDL particle size and LDL particle size is increased; and wherein the risk factor is plasminogen activator inhibitor type-1 (PAI-1), and PAI-1 expression is reduced.

In aspects of the invention utilizing diketopiperazine microparticles, fumaryl diketopiperazine is a preferred type of diketopiperazine, the insulin is dimeric or monomeric, and preferred dosages are up to 90 IU of insulin. In preferred embodiments, inhalation of the dry powder is facilitated by use of a unit dose inhaler.

The present invention will be further understood by reference to the following nonlimiting examples. The following examples make use of Technosphere^{®}/insulin, a proprietary product composed of insulin complexed with fumaryl diketopiperazine microparticles administered as a dry powder aerosol by inhalation.

### Example 1. Pulmonary Delivery of Technosphere^{®}/Insulin to Rats Results in Rapid Absorption.

The pharmacokinetic (PK) profile of pulmonary Technosphere^{®}/insulin particles administered as a dry powder aerosol was compared to the PK profile of human insulin delivered by subcutaneous (s.c.) injection in the rat. A flow-past, nose-only inhalation exposure system was used to administer the aerosols. In the first experiment, all animals received the same formulation (9.1% insulin) but the duration of dosing was adjusted to deliver doses of approximately 1 IU and 3 IU per rat (200 g body weight). A linear dose-dependent response was observed: the maximum serum insulin concentration (C_{MAX}) was 76 ± 12 TIU/mL after a 0.9 IU dose of Technosphere^{®}/insulin and 240 ± 49 TIU/mL after a 2.7 IU dose. The maximum serum insulin levels were obtained in samples taken immediately after the dosing was completed, indicating rapid absorption of Technosphere^{®}/insulin into the systemic circulation. The time to C_{MAX} (T_{MAX}) following inhalation of 0.9 IU Technosphere^{®}/insulin was less than the mean exposure time of 14.5 minutes while the T_{MAx} was 20 minutes for s.c. injection of 1.5 IU. In addition, inhaled Technosphere^{®}/insulin demonstrated a high relative bioavailability of 50 - 70%, compared to s.c. insulin.

In a further experiment, the exposure time was held constant while the insulin content of the Technosphere^{®}/insulin was varied from 2.9 to 11.4% to deliver insulin doses of approximately 0.8 IU, 1.5 IU, and 3 IU. Again, a dose-dependent increase in serum insulin was observed in all groups indicating that the rate of absorption is insensitive to the exact composition of the Technosphere^{®}/insulin powder over this range.

In summary, the precise loading of insulin onto Technosphere^{®} fumaryl diketopiperazine particles and the accurate pulmonary delivery of insulin makes Technosphere^{®}/insulin a non-invasive therapeutic option in the management of diabetes mellitus.

### Example 2. Technosphere^{®} Fumaryl Diketopiperazine Particles Facilitate the Absorption of Insulin in a Primary Cell Culture Model of Alveolar Epithelium Without Evidence of Cytotoxicity.

To investigate the mechanism by which Technosphere^{®}/Insulin product crosses the epithelial barrier of the deep lung, experiments were conducted using monolayers of rat alveolar epithelium in primary culture. Alveolar type II cells were isolated and cultured on semi-permeable polycarbonate membranes until tight monolayers with high trans-epithelial electrical resistance (TEER) were formed. Insulin transport experiments with the Technosphere^{®}/Insulin product and an un-formulated insulin control were then conducted across these monolayers in the apical to basolateral direction at 37°C. Insulin demonstrated an apparent permeability (Pₐₚₚ) of 1.90 ± 0.34 x 10⁻⁸ cm/s, while the Technosphere^{®}/Insulin product demonstrated a Pₐₚₚ that was ten-fold higher at 2.08 ± 0.82 x 10⁻⁷ cm/s. The TEER did not change appreciably between these two groups, or the naïve (untreated) control, indicating that Technosphere^{®} particles do not facilitate the absorption of insulin by disrupting the intercellular tight junctions as calcium chelators do. Apical (donor) well samples were also analyzed for the release of lactate dehydrogenase (LDH), which is a well-established assay for cytotoxicity. LDH activity in the apical media of all groups was less than that of the naive controls (spontaneous LDH release), indicating that Technosphere^{®} particles do not facilitate the absorption of insulin by permeabilizing the cell membrane as non-ionic surfactants and bile salts do. These data indicate that Technosphere^{®}/Insulin product greatly increases the absorption of insulin across the alveolar epithelium without exhibiting any deleterious effects on either intercellular tight junctions or cell membrane integrity.

### Example 3. Treatment of humans with Pulmonary Insulin Reduces Serum Proinsulin Levels.

Inhalation of Technosphere®/Insulin (TI) provides a rise in serum insulin, comparable to the first phase response. This study investigated the pharmacodynamics of TI and its impact on intact proinsulin release, iPi release. Twenty-four patients with Type 2 diabetes received doses of Technosphere® base with 4 different loadings of insulin, either 0, 12 IU, 24 IU or 48 IU of recombinant regular human insulin, five minutes after start of standardized meals, on separate study days. Blood glucose (BG), serum insulin and serum iPi were measured before (0 min), 60 and 120 min after initiation of each meal.

TI lowered postprandial BG levels in a dose-dependent manner. Sixty minutes after lunch, BG (mg/dl) (± SD) was 183.2 (± 44.4) for placebo; 170.8(± 30.5) for 12 IU (p= 0.266); 156.3(± 31.9) for 24 IU, (p= 0.020) and 132.6(± 29.1) for 48 IU, (p< 0.001). All doses caused an increase in serum insulin at 60 minutes (p<0.05), but not at 120 minutes following inhalation. Administration of TI with 24 IU and 48 IU insulin load doses suppressed iPi levels at all time points throughout the day (p< 0.05) (Fig 1).

The use of inhaled TI to mimic the rapid onset and short duration of the first phase insulin response therefore should reduce postprandial stress on the beta cell population. This can improve general beta cell function and endogenous glucose homeostasis

## Claims

1. A dose of insulin that is effective to reduce serum proinsulin levels and which mimics a first phase insulin response, for use as a prandial medicament for reducing risk factors for atherosclerotic cardiovascular disease in a patient who is in the early course of type 2 diabetes.

2. The dose of insulin for use according to Claim 1 wherein the dose of the insulin is sufficient to control blood glucose levels.

3. The dose of insulin for use according to Claim 1 wherein a dose of the insulin is sufficient to control blood glucose levels, whereby pancreatic stress is attenuated.

4. The dose of insulin for use according to Claim 1, wherein the risk factor is LDL particle size and LDL particle size is increased, or the risk factor is plasminogen activator inhibitor type-1 (PAI-1) and PAI-1 expression is reduced.

5. The dose of insulin for use according to Claim 1 wherein the dose of the insulin is sufficient to shut off gluconeogenesis.

6. The dose of insulin for use according to Claim 1 wherein the insulin is administered within 10 minutes after starting a meal.

7. The dose of insulin for use according to Claim 1 wherein the insulin is administered as a pulmonary or dry powder formulation.

8. The dose of insulin for use according to Claim 7 wherein the formulation is a diketopiperazine microparticle drug delivery system.

9. The dose of insulin for use according to Claim 8 wherein the diketopiperazine is fumaryl diketopiperazine.

10. The dose of insulin for use according to Claim 9 wherein the insulin is administered by pulmonary delivery as biodegradable polymeric or surfactant microparticles incorporating the insulin.

11. The dose of insulin for use according to Claim 1 wherein the insulin is dimeric or monomeric.

12. The dose of insulin for use according to Claim 1 wherein a dose of the insulin is up to 90 IU.

13. The dose of insulin for use according to Claim 12 wherein the dose is up to 48 IU.

14. The dose of insulin for use according to Claim 1 wherein serum insulin levels peak within 18 minutes of administration

15. The dose of insulin for use according to Claim 1 wherein serum insulin levels return to baseline within 2 hours of administration.

16. The dose of insulin for use according to Claim 1 wherein the insulin is administered within one hour after starting a meal.

17. Use of a dose of insulin that is effective to reduce serum proinsulin levels and which mimics a first phase insulin response, in the manufacture of a prandial medicament for reducing risk factors for atherosclerotic cardiovascular disease in a patient who is in the early course of type 2 diabetes.

## Patentansprüche

1. Insulindosis, die wirksam ist, Serumproinsulinspiegel zu verringern, und die eine Anfangsphasen-Insulinantwort nachahmt, zur Verwendung als ein prandiales Medikament zum Verringern von Risikofaktoren für eine atherosklerotische kardiovaskuläre Erkrankung in einem Patienten, der im Frühverlauf von Typ-2-Diabetes ist.

2. Insulindosis zur Verwendung nach Anspruch 1, wobei die Dosis des Insulins ausreichend ist, um Blutzuckerwerte zu kontrollieren.

3. Insulindosis zur Verwendung nach Anspruch 1, wobei eine Dosis des Insulins ausreichend ist, um Blutzuckerwerte zu kontrollieren, wobei die Belastung der Bauchspeicheldrüse abgeschwächt ist.

4. Insulindosis zur Verwendung nach Anspruch 1, wobei der Risikofaktor LDL-Partikelgröße ist und LDL-Partikelgröße vergrößert ist, oder der Risikofaktor PlasminogenAktivator-Hemmer Typ 1 (PAI-1) ist und die PAI-1-Exprimierung verringert ist.

5. Insulindosis zur Verwendung nach Anspruch 1, wobei die Dosis des Insulins ausreichend ist, um Gluconeogenese auszuschalten.

6. Insulindosis zur Verwendung nach Anspruch 1, wobei das Insulin innerhalb von 10 Minuten nach dem Beginn einer Mahlzeit verabreicht wird.

7. Insulindosis zur Verwendung nach Anspruch 1, wobei das Insulin als eine pulmonale oder Trockenpulverformulierung verabreicht wird.

8. Insulindosis zur Verwendung nach Anspruch 7, wobei die Formulierung ein Diketopiperazin-Mikropartikel-Arzneimittelabgabesystem ist.

9. Insulindosis zur Verwendung nach Anspruch 8, wobei das Diketopiperazin Fumaryldiketopiperazin ist.

10. Insulindosis zur Verwendung nach Anspruch 9, wobei das Insulin durch pulmonale Verabreichung als biologisch abbaubare polymere oder oberflächenaktive Mikropartikel, die das Insulin enthalten, verabreicht wird.

11. Insulindosis zur Verwendung nach Anspruch 1, wobei das Insulin dimer oder monomer ist.

12. Insulindosis zur Verwendung nach Anspruch 1, wobei eine Dosis des Insulins bis zu 90 IE beträgt.

13. Insulindosis zur Verwendung nach Anspruch 12, wobei die Dosis bis zu 48 IE beträgt.

14. Insulindosis zur Verwendung nach Anspruch 1, wobei Seruminsulinspiegel innerhalb von 18 Minuten nach dem Verabreichen den Spitzenwert erreichen.

15. Insulindosis zur Verwendung nach Anspruch 1, wobei Seruminsulinspiegel innerhalb von 2 Stunden nach dem Verabreichen zum Ausgangswert zurückkehren.

16. Insulindosis zur Verwendung nach Anspruch 1, wobei das Insulin innerhalb von einer Stunde nach dem Beginn einer Mahlzeit verabreicht wird.

17. Verwendung einer Insulindosis, die wirksam ist, Serumproinsulinspiegel zu verringern, und die eine Anfangsphasen-Insulinantwort nachahmt, beim Herstellen eines prandialen Medikaments zum Verringern von Risikofaktoren für eine atherosklerotische kardiovaskuläre Erkrankung in einem Patienten, der im Frühverlauf von Typ-2-Diabetes ist.

## Revendications

1. Dose d'insuline efficace pour réduire les taux sériques de proinsuline et qui reproduit une réaction à l'insuline de première phase, pour une utilisation en tant que médicament prandial pour réduire les facteurs de risque applicables à une maladie cardiovasculaire athéroscléreuse chez un patient atteint de diabète de type 2 au stade précoce.

2. Dose d'insuline pour une utilisation selon la revendication 1, laquelle dose d'insuline est suffisante pour contrôler les taux de glycémie.

3. Dose d'insuline pour une utilisation selon la revendication 1, laquelle dose d'insuline est suffisante pour contrôler les taux de glycémie, par laquelle le stress pancréatique est atténué.

4. Dose d'insuline pour une utilisation selon la revendication 1, dans laquelle le facteur de risque est la taille des particules LDL et la taille des particules LDL est augmentée, ou le facteur de risque est l'inhibiteur de l'activateur du plasminogène de type 1 (PAI-1) et l'expression de PAI-1 est réduite.

5. Dose d'insuline pour une utilisation selon la revendication 1, laquelle dose d'insuline est suffisante pour bloquer la gluconéogenèse.

6. Dose d'insuline pour une utilisation selon la revendication 1, dans laquelle l'insuline est administrée dans les 10 minutes qui suivent le début d'un repas.

7. Dose d'insuline pour une utilisation selon la revendication 1, dans laquelle l'insuline est administrée sous forme de formulation administrable par voie pulmonaire ou de poudre sèche.

8. Dose d'insuline pour une utilisation selon la revendication 7, dans laquelle la formulation est un système d'administration de médicaments contenant des microparticules de dicétopipérazine.

9. Dose d'insuline pour une utilisation selon la revendication 8, dans laquelle la dicétopipérazine est la dicétopipérazine de fumaryle.

10. Dose d'insuline pour une utilisation selon la revendication 9, dans laquelle l'insuline est administrée par voie pulmonaire sous forme de microparticules de polymères ou de tensioactifs biodégradables incorporant l'insuline.

11. Dose d'insuline pour une utilisation selon la revendication 1, dans laquelle l'insuline est dimérique ou monomérique.

12. Dose d'insuline pour une utilisation selon la revendication 1, laquelle dose d'insuline atteint 90 UI.

13. Dose d'insuline pour une utilisation selon la revendication 12, laquelle dose atteint 48 Ul.

14. Dose d'insuline pour une utilisation selon la revendication 1, dans laquelle les taux sériques d'insuline atteignent leur niveau maximal dans les 18 minutes qui suivent l'administration.

15. Dose d'insuline pour une utilisation selon la revendication 1, dans laquelle les taux sériques d'insuline retrouvent leur valeur de base dans les 2 heures qui suivent l'administration.

16. Dose d'insuline pour une utilisation selon la revendication 1, dans laquelle l'insuline est administrée dans l'heure qui suit le début d'un repas.

17. Utilisation d'une dose d'insuline efficace pour réduire les taux sériques de proinsuline et qui reproduit une réaction à l'insuline de première phase, dans la fabrication d'un médicament prandial pour réduire les facteurs de risque applicables à une maladie cardiovasculaire athéroscléreuse chez un patient atteint de diabète de type 2 au stade précoce.
